# EUROPEAN PATENT APPLICATION

(11) **EP 0 908 836 A2**
(43) Date of publication of application: **14.04.1999**
(21) Application number: 98308141.5
(22) Date of filing: 06.10.1998
(51) Int. Cl.: G06F 19/00, A61B 17/17

(54) **Computer-constructed surgical guide**

(30) Priority: 06.10.1997 US 944275
(71) Applicant: GENERAL ELECTRIC COMPANY, Schenectady, NY 12345 (US)
(72) Inventor: Vomlehn, John Christian, Scotia, New York 12302 (US); Vosburgh, Kirby Gannett, Schenectady, New York 12309 (US); Carl, Allen Lawrence, Slingerlands, New York 12159 (US)
(74) Representative: Goode, Ian Roy

(57) **Abstract**

A system for constructing a reference structure intended to fit flush against an anchor site of a subject is used to anchor and guide medical equipment, or used for accurate placement of fasteners into the anchor site. The present invention employs a medical imaging device (11) which acquires data of internal structures of the subject. This data is segmented (19) into discrete solid structures. These solid structures are displayed on a user interface (23) as a 3D computer model. The user then selects a structure and an anchor site on the structure which a fastener is to be positioned. The user may also interactively indicate, through the user interface, a location and orientation in which the fastener is to be inserted. A design device (25) creates a surgical guide having a mating face which fits flush against the anchor site. The guide may have pre-drilled guide holes for receiving a surgical instrument, such as a drill for drilling into the anchor site. It may have an attachment structure for attaching medical equipment. Several probe holes may also be made which receive a probe and intersect with the anchor site. Markings on the probe indicate if the mating face is flush against the anchor site.

## Description

The present invention relates to computer-aided construction of a reference structure to be attached to a subject and act as a guide in medical procedures.

In various medical procedures, it is necessary to attach a piece of medical equipment into a solid structure of the subject.

Typically, screws are inserted into vertebra on both ends of a structurally-compromised vertebrae to provide support while a spinal fusion is being created. Fractures in the spine and in other bones are also held together with a screw or similar implant.

In the case of abnormal spinal structure, it may necessary to fasten a support structure to correct or straighten a subject's spine.

These examples require precise insertion of a screw or pin into bone of the subject.

Typically, these pins or screws have been inserted by a surgeon who visually, or by 'feel', finds the approximate location where the screw or pin should be entered, and drills a hole at that location. The screw or pin is inserted into the hole.

Sometimes, during surgery, two dimensional (2D) snapshots such as x-rays or magnetic resonance (MR) images may be obtained.

Since these are 2D in nature, it may be difficult to extrapolate the image plane to the plane allowing the access of insertion of the screw.

Since the surgeon would also like to make as small an incision as possible, many times the target area is obscured by muscle or other tissue and the surgeon approximates the location of the actual target point.

Location of the target location may be further obscured by blood profusion in the area, further complicating identification of the target point.

Once the approximate location is determined, the surgeon has little information as to the relative strength and thickness of the bone in the area in which the pin or screw is to be inserted.

Also, while applying these screws and pins, it is possible that the surgeon punctures or severs major arteries, veins, or nerves which may be hidden inside tissue over the target structure, or lie behind the target structure, not visible to the surgeon.

Currently there is a need for a device which may be attached to a subject and act as a reference structure to guide instruments during medical procedures.

The present invention constructs a reference structure intended to be attached to a solid anchor site of a subject.

A medical imaging means acquires medical imaging data of the subject 1 in a region including the anchor site. The user may operate a user interface to steer the medical imaging means to the proper region.

A segmentation device identifies a segmented structure being contiguous locations in the imaging data having the data values within a defined range.

A graphic engine rendering surfaces of the segmented structures according to user-defined imaging parameters.

A design device, which may be a conventional computer aided design (CAD) device, creates a computer model of a reference structure having a mating surface designed to fit flush with said subject's anchor site.

In an alternative embodiment of the present invention, an actuator device mills a surgical guide according to the design of the design device.

The guide may have an attachment structure capable of attaching to other medical equipment. This equipment may then be anchored to, and use the reference structure as a landmark, to determine locations and perform medical procedures.

It is an object of the present invention to create a reference structure which physically guides surgical instruments.

It is another object of the present invention create a more accurate means of performing surgical procedures.

The features of the invention believed to be novel are set forth with particularity in the appended claims. The invention itself, however, both as to organization and method of operation, together with further objects and advantages thereof, may be best understood by reference to the following description taken in conjunction with the accompanying drawing in which:

Figure 1 is a simplified block diagram of one embodiment of the present invention in operation tracking the location of a surgical device relative to a desired location within a subject.

Figure 2 is a an illustration of the computer-constructed reference structure attached to an anchor site of a subject and a surgical instrument functioning with the reference structure.

In order to properly operate a medical device on a subject, it must be stable, and located at a proper position and orientation, (pose). The use of a reference structure would be very helpful in anchoring or guiding the equipment. This reference structure should attach to a solid structure of the subject in such a manner that it fits without movement, and is able to direct the medical instrument in a precise manner.

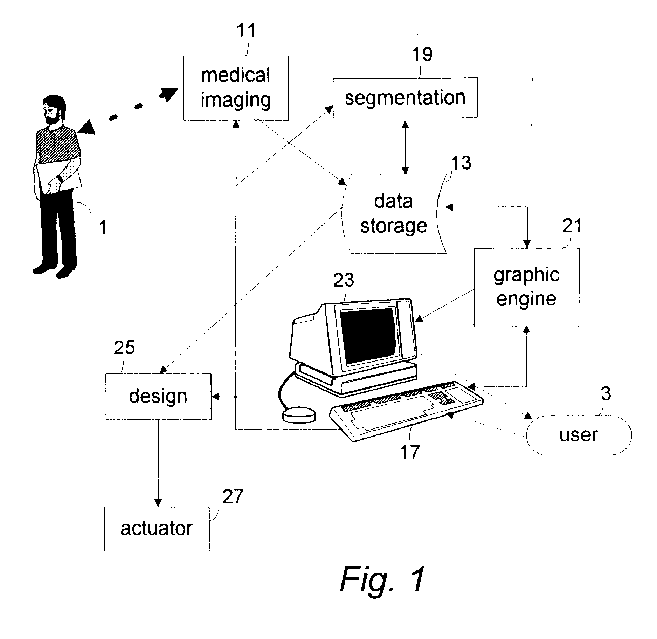

The present invention, as shown in Figure 1, operates to construct such a reference structure.

A subject 1 on which the procedure is to be performed, is imaged with a medical imaging device 11. Medical imaging device 11 may be a computed tomography (CT), magnetic resonance (MR), ultrasound, or positron emission tomography (PET) imaging device. Other types of medical imaging device may also be used which provide an image of internal organs of subject 1 and can provide an image of tracked targets 28.

A user 3, which preferably is a surgeon, operates medical imaging device 11 through a user interface 17 to select the proper portion of subject 1 to be viewed. Volumetric data from medical imaging device 11 is passed to and stored in memory device 13. This may physically be part of medical imaging device 11 or be a separate device.

A segmentation device 19 interacts with the volumetric data stored in data storage device 13 and determines data values within a range which may interactively be defined by user 3 via interface 17. These values are used to define a tissue type. Contiguous locations having the same tissue type are then determined. The set of all contiguous locations of the same tissue type are treated as a solid object or structure. This information may be stored in segmentation device 19 or data storage device 13.

Conventional segmentation may be used, such as that described in U.S. patent 5,187,658, February 16, 1993 by Cline, and Lorensen.

User 3 interactively indicates solid structures of the subject 1 to be displayed using user interface 17. User 3 may also indicate the viewpoint, degree of transparency of different surfaces, color coding of different structures, and other graphic parameters used in displaying images on a monitor 23.

Graphic engine 21 interacts with the volumetric data in memory device 13 to determine surfaces of a user-defined segmented structure which the user 3 indicates should be displayed on monitor 23. For example, user 3 may select a mode in which layers are stripped away and use a pointing device, such as a mouse, to indicate structures which user 3 would like removed from the visual display. This allows underlying structures to be seen. Any conventional surface rendering technique and workstation may be used.

User 3 also identifies an anchor site through user interface 17 interacting with graphics engine 21, which is a solid structure, typically bone, onto which reference structure 30 is attached.

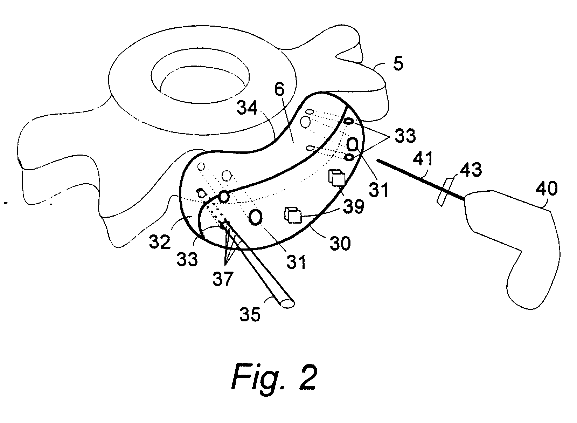

A design device 25, which may be a computer aided design (CAD) device, interacts with user 3 through user interface 17 to 'build' a reference structure 30, as shown in Fig. 2, having a mating surface 34, designed to fit flush against the surface of the solid structure at anchor site 6 of the subject. Design device 25 may be any conventional CAD device which allows input of other models.

Reference structure 30 may then be physically held against an anchor site 6 by the surgeon, or attached by various conventional means such as screws, pins, glue, clamps, etc. The reference structure 30 may then be used as a reference, or anchor structure for other surgical equipment.

In one embodiment, an attachment structure 39 functions to allow other medical equipment to attach to it. This equipment may then use reference structure 30 as a landmark to determine locations relative to reference structure 30. It can then act to steady and guide drills, probes, and other interventional devices.

In an optional embodiment, user 3 also may use a pointing device of user interface 17 to select a position and orientation (pose) which in a surgical instrument 40 is positioned in order to correctly insert the screw or pin. Design device 25 or graphics engine 21 may have a computer model of surgical instrument 40 pre-stored, and superimpose this model upon the images provided on monitor 23.

User 3 may then interactively adjust the pose of surgical instrument 40 by viewing its computer graphic model such that the proper pose is indicated on monitor 23. This pose may be saved and incorporated as a guide hole.

The final pose of surgical instrument may be used to construct a guide hole 31. Guide hole 31 allows a shaft 41 of medical equipment, such as a surgical drill 40, to fit through snugly with little clearance, intended to restrain motion of the drill 40 along in all directions except along an axis of guide hole 31.

Design device 25 develops a computer model of reference structure 30 which may be output to an actuator system 27. Actuator system 27 mills and drills a biocompatible material to form reference structure 30. A biocompatible material are those commonly used as medical implants, which are not poisonous, and do not cause rejections or immune-response reactions. Design device 25 may alternatively output the design of reference structure 30 to actuator 27 for later off-line manufacture.

A collar 43 may be attached to surgical instrument 40 at the intended depth to be used as a stop when drilling through reference structure 30. This restrains motion along the axis of the guide hole at a predetermined depth.

Alternatively, reference structure 30 may be constructed to a height which stops surgical instrument 40 when instrument shaft 41 is at its proper depth.

Reference structure 30 is intended to be held flush against solid structure 5, however, there are times that it may not mate correctly with solid structure 5. In an alternative embodiment, probe holes 33 are drilled through a body 32 of reference structure 30. A depth probe 35 is positioned within probe holes 33. Depth probe 35 is pushed inward until it contacts anchor site 6 of solid structure 5. By markings or indication on depth probe 35, it can be discerned if reference structure 30 is positioned flush with respect to solid structure 5. This also allows for readjustment and proper placements of image guide 30.

## Claims

1. A reference structure construction system, for constructing reference structure (30) for aiding in anchoring and guiding medical equipment at an anchor site (6) of a subject (1) comprising:
a) a data storage device (13) capable of storing imaging data;
b) medical imaging means (11) coupled to the data storage device (11) for acquiring medical imaging data of said subject (1) according to predetermined or user-defined imaging parameters of a region including said anchor site (6) and for storing the data in the data storage device (13);
c) a segmentation device (19) coupled to the data storage device (13) for identifying contiguous locations in the imaging data having the data values within a predetermined or user-defined range indicating the same tissue type, being a segmented structure and for identifying surfaces between tissue types;
d) a graphic engine (21) coupled to the data storage device (13) for rendering surfaces of the segmented structures according to predetermined or user-defined imaging parameters;
e) a design device (25) coupled to the data storage device (13) for creating a computer model according to predetermined or user-defined parameters of a surgical guide (30) having a mating surface (34) designed to fit flush with said subject's anchor site (6).

2. The reference structure construction system recited in claim 1, further comprising:
a user interface (17) coupled to the medical imaging means (11), the segmentation device (19), and the design device (25) for providing user-defined parameters to these elements.

3. The reference structure construction system recited in claim 1, further comprising:
an actuator device (27) coupled to design device (25) for milling a surgical guide according to the computer model constructed by the design device (25).

4. A reference structure assembly for anchoring and guiding medical equipment at an anchor site (6) of a subject 1 comprising:
a) a body (32) having a mating surface (34) capable of fitting flush against said anchor site (6);
b) at least one probe hole (33) passing through the body (32) intersecting the anchor site (6);
c) at least one elongated probe (35) passing through the probe hole (31), and resting on the anchor site (6), having markings (37) measuring the depth of which the probe (35) fits into the body (30), indicating a separation between the anchor site (6) and the mating surface (34).

5. The reference structure assembly of claim 4 further comprising: at least one guide hole (31) through the body (32) for receiving a portion of the medical equipment, passing through the body (32) at a position and orientation to intersect the anchor site (6) when the mating surface (34) is flush against the anchor site (6).

6. The reference structure assembly of claim 4 further comprising: a collar designed to attach to a portion of a medical equipment intended to be received by the guide hole, thereby restricting the depth in which the surgical instrument is inserted into the guide hole.
